# EUROPEAN PATENT APPLICATION

(11) **EP 4 434 969 A1**
(43) Date of publication of application: **25.09.2024**
(21) Application number: 22892930.3
(22) Date of filing: 15.11.2022
(51) Int. Cl.: C07D 221/12, A61K 31/473, A61P 31/14, C07D 401/12, C07D 405/12

(54) **ANTI-SARS-COV-2 DRUG**

(30) Priority: 15.11.2021 JP 2021185802
(71) Applicant: Oncolys BioPharma, Inc., Tokyo 105-0001 (JP)
(72) Inventor: BABA, Masanori, Kagoshima-shi, Kagoshima 890-8580 (JP); OKAMOTO, Mika, Kagoshima-shi, Kagoshima 890-8580 (JP); TOYAMA, Masaaki, Kagoshima-shi, Kagoshima 890-8580 (JP); HASHIMOTO, Hiromasa, Takatsuki-shi, Osaka 569-0084 (JP)
(74) Representative: J A Kemp LLP
(86) International application number: PCT/JP2022/042332
(87) International publication number: WO 2023/085432

(57) **Abstract**

The present invention pertains to: a compound represented by formula (I): 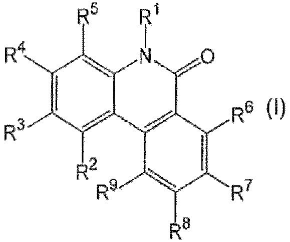 [in the formula, R¹ represents C₁-C₈ alkyl or the like, R²-R⁹ each independently represent hydrogen, a halogen, hydroxyl, NH₂, Q-(C₁-C₈ alkyl) (Q represents O, NH, or S), or the like, and at least one of R⁷ and R⁸ represents NH₂ or NH(R¹⁰) (R¹⁰ represents substituted or unsubstituted C₁-C₈ alkyl or the like)], a pharmaceutically acceptable salt thereof, a solvate thereof, or a prodrug thereof; and use of those as an anti-coronavirus drug.

## Description

### Technical Field

The present invention relates to an anti-coronavirus drug.

### Background Art

Coronaviruses are viruses which essentially cause cold symptoms in humans. Four types of coronaviruses have been known, and 10 to 15% of colds are caused by these viruses. In addition, coronaviruses causing Severe Acute Respiratory Syndrome (SARS) and Middle East Respiratory Syndrome (MERS), both having a high fatality rate, have been known so far. The number of patients with SARS is approximately 8,000 with a fatality rate of approximately 10% and the number of patients with MERS is approximately 2,500 with a fatality rate of approximately 35%.

Coronaviruses are positive-stranded RNA viruses having an envelope of approximately 100 nm in diameter. SARS-CoV is classified as a Class II pathogen and MERS-CoV is classified as a Class III pathogen.

Various antibody drugs targeting the spike protein on the surface of SARS-CoV-2, and remdesivir, diverted from an anti-Ebola drug, have been used as therapeutic agents at present. In addition, molnupiravir, developed as an anti-influenza drug, and Paxlovid, newly developed, have been approved as orally administrable anti-SARS-CoV-2 drugs. It is deemed that molnupiravir inhibits viral RNA-dependent RNA polymerases, and causes errors in the replication of virus RNAs in the action mechanism thereof. Paxlovid is a drug combination of nirmatrelvir, which is a low molecule compound which inhibits the functions of the main proteases necessary for the replication of viruses, and ritonavir, which functions as a booster for maintaining the blood level thereof. However, these drugs have some disadvantages in that the drugs have side effects such as teratogenicity, and the combined use of the drugs and other drugs is problematic. Accordingly, it is very important to identify and develop a new drug having selective and strong antivirus effects on SARS-CoV-2.

Meanwhile, the present inventors have found that phenanthridinone derivatives are effective against human hepatitis C virus and filed a patent application (Patent Literature 1).

However, the relationship between phenanthridinone derivatives and their anti-coronavirus activity has not been reported so far.

### Citation List

### Patent Literature

[Patent Literature 1] WO 2011/093483

### Summary of Invention

### Technical Problem

An object of the present invention is to provide an antiviral agent which is effective against coronaviruses such as SARS-CoV-2.

### Solution to Problem

To attain the above object, the present inventors have established an anti-coronavirus assay system for agents and proceeded with screening of various agents. As a result, they have found anti-coronavirus effects against coronaviruses such as SARS-CoV-2 in specific phenanthridinone derivatives, thus completing the present invention.

Specifically, the present invention is summarized as follows.
(1) A compound represented by formula (I), a pharmaceutically acceptable salt thereof, a solvate thereof, or a prodrug thereof 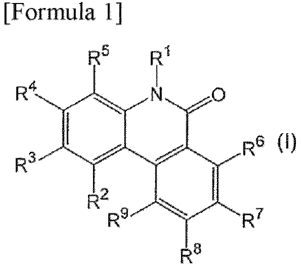 wherein,
   R¹ is selected from C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from halogen, hydroxyl, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(Ci-Cs alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)); and
   R² to R⁹ are each independently selected from Substituent group A consisting of hydrogen, halogen, hydroxyl, NH₂, NO₂, OSO₃H, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), NH-C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), NH-C(NH)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), N(R¹⁰)(R¹¹) (wherein R¹⁰ and R¹¹ are each independently C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl), NH(R¹⁰) (wherein R¹⁰ is C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl), Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl) and Q-(heteroarylalkyl) (wherein Q is O, NH, or S), Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from halogen, hydroxyl, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), NH-C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), N(R¹⁰)(R¹¹) (wherein R¹⁰ and R¹¹ are each independently C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)),
   provided that any two of R² to R⁵ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from halogen, hydroxyl, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)),
   at least one of R⁷ and R⁸ is NH₂, NH-C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), NH-C(NH)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), N(R¹⁰)(R¹¹) (wherein R¹⁰ and R¹¹ are each independently C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl), or NH(R¹⁰) (wherein R¹⁰ is C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl) (each of these groups being independently unsubstituted or substituted with one or more groups selected from halogen, hydroxyl, NH₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), NH-C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), N(R¹⁰)(R¹¹) (wherein R¹⁰ and R¹¹ are each independently C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)).
(2) The compound according to (1), a pharmaceutically acceptable salt thereof, a solvate thereof, or a prodrug thereof, wherein at least one of R⁷ and R⁸ is NH₂, NH-C(O)Z (wherein Z is C₁-C₈ alkyl, heterocyclyl, or NH-(C₁-C₈ alkyl), and these groups are unsubstituted or substituted with one or more groups selected from hydroxyl and NH₂), N(R¹⁰)(R¹¹) (wherein R¹⁰ and R¹¹ are each independently C₁-C₈ alkyl or arylalkyl, and these groups are unsubstituted or substituted with one or more groups selected from hydroxyl, O-(C₁-C₈ alkyl), NH₂, N(CH₃)₂, and CONH₂), or NH(R¹⁰) (wherein R¹⁰ is C₁-C₈ alkyl, arylalkyl, or heterocyclyl, and these groups are unsubstituted or substituted with one or more groups selected from hydroxyl, O-(C₁-C₈ alkyl), NH₂, N(CH₃)₂, and CONH₂).
(3) The compound according to (1), a pharmaceutically acceptable salt thereof, a solvate thereof, or a prodrug thereof, wherein R⁸ is NH(R¹⁰) (wherein R¹⁰ is C₂-C₅ alkyl substituted with one or two hydroxyl), or NH-C(O)Z (wherein Z is heterocyclyl substituted with one hydroxyl).
(4) The compound according to any one of (1) to (3), a pharmaceutically acceptable salt thereof, a solvate thereof, or a prodrug thereof, wherein R¹ is C₃-C₇ alkyl.
(5) The compound according to any one of (1) to (3), a pharmaceutically acceptable salt thereof, a solvate thereof, or a prodrug thereof, wherein R¹ is C₄ alkyl.
(6) The compound according to any one of (1) to (5), a pharmaceutically acceptable salt thereof, a solvate thereof, or a prodrug thereof, wherein R³ is 1,1,1,3,3,3-hexafluoro-2-hydroxypropan-2-yl.
(7) An anti-coronavirus drug comprising, as an active ingredient, the compound according to any one of (1) to (6), a pharmaceutically acceptable salt thereof, a solvate thereof, or a prodrug thereof.
(8) The anti-coronavirus drug according to (7), which is an anti-SARS-CoV-2 drug.
(9) The anti-coronavirus drug according to (8), which is used for preventing and/or treating COVID-19.
(10) Use of the compound according to any one of (1) to (6), a pharmaceutically acceptable salt thereof, a solvate thereof, or a prodrug thereof for the manufacture of an anti-coronavirus drug.
(11) The use according to (10), wherein the anti-coronavirus drug is an anti-SARS-CoV-2 drug.
(12) The use according to (10), wherein the anti-coronavirus drug is used for preventing or treating COVID-19.

### Advantageous Effects of Invention

The present invention can provide an antiviral agent which is effective against SARS-CoV-2.

### Description of Embodiments

Hereafter, preferable embodiments of the present invention are described in detail.

The term "alkyl" used herein refers to a linear or branched aliphatic hydrocarbon group having a specific number of carbon atoms. For example, the term "Ci-Cs alkyl" refers to a linear or branched hydrocarbon chain having at least 1 and up to 8 carbon atoms. Examples of alkyl that can be preferably used include, but are not limited to, methyl, ethyl, n-propyl, isopropyl, n-butyl, sec-butyl, isobutyl, tert-butyl, n-pentyl, n-hexyl, n-heptyl, and n-octyl.

The term "alkenyl" used herein refers to a group resulting from substitution of one or more C-C single bonds of the alkyl with double bonds. Examples of preferable alkenyl include, but are not limited to, vinyl, 1-propenyl, allyl, 1-methylethenyl(isopropenyl), 1-butenyl, 2-butenyl, 3-butenyl, 1-methyl-2-propenyl, 2-methyl-2-propenyl, 1-methyl-1-propenyl, 2-methyl-1-propenyl, 1-pentenyl, 1-hexenyl, n-heptenyl, and 1-octenyl.

The term "alkynyl" used herein refers to a group resulting from substitution of one or more C-C single bonds of the alkyl with triple bonds. Examples of preferable alkynyl include, but are not limited to, ethynyl, 1-propinyl, 2-propinyl, 1-butynyl, 2-butynyl, 3-butynyl, 1-methyl-2-propinyl, 1-pentynyl, 1-hexynyl, 1-heptynyl, and 1-octynyl.

The term "cycloalkyl" used herein refers to alicyclic alkyl having a specific number of carbon atoms. For example, the term "C₃-C₆ cycloalkyl" refers to a cyclic hydrocarbon group having at least 3 and up to 6 carbon atoms. Examples of preferable cycloalkyl include, but are not limited to, cyclopropyl, cyclobutyl, cyclopentyl, and cyclohexyl.

The term "cycloalkenyl" used herein refers to a group resulting from substitution of one or more C-C single bonds of the cycloalkyl with double bonds. Examples of preferable cycloalkenyl include, but are not limited to, cyclopropenyl, cyclobutenyl, cyclopentenyl, and cyclohexenyl.

The term "cycloalkynyl" used herein refers to a group resulting from substitution of one or more C-C single bonds of the cycloalkyl with triple bonds. Examples of preferable cycloalkynyl include, but are not limited to, cyclobutynyl, cyclopentynyl, and cyclohexynyl.

The term "heterocyclyl" used herein refers to a group resulting from substitution of one or more carbon atoms of the cycloalkyl, cycloalkenyl, or cycloalkynyl with a hetero atom or hetero atoms selected from nitrogen (N), sulfur (S), and oxygen (O). In this case, substitution with N or S includes substitution with N- or S-oxide or dioxide. Examples of preferable heterocyclyl include, but are not limited to, pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, and piperazinyl.

The term "aryl" used herein refers to an aromatic ring group having 6 to 15 carbon atoms. Examples of preferable aryl include, but are not limited to, phenyl, naphthyl, and anthryl (anthracenyl).

The term "arylalkyl" used herein refers to a group resulting from substitution of a hydrogen atom of the alkyl with the aryl. Examples of preferable arylalkyl include, but are not limited to, benzyl, 1-phenethyl, and 2-phenethyl.

The term "arylalkenyl" used herein refers to a group resulting from substitution of a hydrogen atom of the alkenyl with the aryl. An example of preferable arylalkenyl is, but is not limited to, styryl.

The term "heteroaryl" used herein refers to a group resulting from substitution of one or more carbon atoms of the aryl with a hetero atom or hetero atoms selected from nitrogen (N), sulfur (S), and oxygen (O). In this case, substitution with N or S includes substitution with Nor S-oxide or dioxide. Examples of preferable heteroaryl include, but are not limited to, furanyl, thienyl, pyrrolyl, imidazolyl, pyrazolyl, triazolyl, tetrazolyl, thiazolyl, oxazolyl, isooxazolyl, oxadiazolyl, thiadiazolyl, isothiazolyl, pyridyl, pyridazinyl, pyrazinyl, pyrimidinyl, quinolinyl, isoquinolinyl, and indolyl.

The term "heteroarylalkyl" used herein refers to a group resulting from substitution of a hydrogen atom of the alkyl with the heteroaryl.

The groups described above can be each independently unsubstituted or substituted with one or more groups selected from halogen, hydroxyl, NH₂, NO₂, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), NH-C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), N(R¹⁰)(R¹¹) (wherein R¹⁰ and R¹¹ are each independently C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S).

The term "halogen" or "halo" used herein refers to fluorine, chlorine, bromine, or iodine.

The term "salt" used herein preferably refers to a pharmaceutically acceptable salt. In this case, examples of preferable counter ions of a compound represented by formula (I) include, but are not limited to, cations such as sodium ions, potassium ions, calcium ions or magnesium ions, or anions such as chloride ions, bromide ions, formate ions, acetate ions, maleate ions, fumarate ions, benzoate ions, ascorbate ions, pamoate ions, succinate ions, bis-methylenesalicylate ions, methanesulfonate ions, ethane disulfonate ions, propionate ions, tartrate ions, salicylate ions, citrate ions, gluconate ions, aspartate ions, stearate ions, palmitate ions, itaconate ions, glycolate ions, p-aminobenzoate ions, glutamate ions, benzenesulfonate ions, cyclohexylsulfamate ions, methanesulfonate ions, ethanesulfonate ions, isethionate ions, benzenesulfonate ions, p-toluenesulfonate ions, naphthalenesulfonate ions, phosphate ions, nitrate ions, sulfate ions, carbonate ions, bicarbonate ions, and perchlorate ions.

Examples of a solvate of the compound represented by formula (I), or the salt thereof include a hydrate.

The compound represented by formula (I), the salt thereof, or the solvate thereof may be a deuterium converter obtained by converting ¹H to ²H (D). Such a compound is also included in the present invention.

In the specification, the "prodrug" refers to any compound as long as the compound, when administered to a living organism, generates the compound of formula (I) through a spontaneous chemical reaction, or a catalytic enzyme or a metabolic reaction. Examples of groups constituting a prodrug used for a hydroxyl group or amino group include a C₂₋₇-acyl group, a C₁₋₆-alkoxy(C₂₋₇-acyl) group, a C₁₋₆-alkoxycarbonyl(C₂₋₇-acyl) group, a C₁₋₆-alkoxycarbonyl group, a C₁₋₆-alkoxy(C₂₋₇-alkoxycarbonyl) group, a (C₂₋₇-acyloxy)methyl group, a 1-(C₂₋₇-acyloxy)ethyl group, a (C₂₋₇-alkoxycarbonyl) oxymethyl group and a 1-[(C₂₋₇-alkoxycarbonyl)oxy]ethyl group, and a C₂₋₇-acyl group and a C₁₋₆-alkoxycarbonyl group are preferable. Examples of groups constituting a prodrug used for a carboxyl group include a C₁₋₆-alkyl group, a C₁₋₆-alkoxy-C₁₋₆-alkyl group, a (C₂₋₇-acyloxy)methyl group, a 1-(C₂₋₇-acyloxy)ethyl group, a (C₂₋₇-alkoxycarbonyl)oxymethyl group and a 1-[(C₂₋₇-alkoxycarbonyl)oxy]ethyl group, and a C₁₋₆-alkyl group and a C₁₋₆-alkoxy-C₁₋₆-alkyl group are preferable.

The compound represented by formula (I), the pharmaceutically acceptable salt thereof, the solvate thereof, or the prodrug thereof also includes stereoisomers such as racemates and optically active forms.

In the compound represented by formula (I), R¹ is preferably C₃-C₇ alkyl, more preferably C₄ alkyl, and R³ is preferably 1,1,1,3,3,3-hexafluoro-2-hydroxypropan-2-yl.

In the compound represented by formula (I), a compound in which at least one of R⁷ and R⁸ is NH₂, NH-C(O)Z (wherein Z is Ci-Cs alkyl, heterocyclyl, or NH-(Ci-Cs alkyl), and these groups are unsubstituted or substituted with one or more groups selected from hydroxyl and NH₂), N(R¹⁰)(R¹¹) (wherein R¹⁰ and R¹¹ are each independently C₁-C₈ alkyl or arylalkyl, and these groups are unsubstituted or substituted with one or more groups selected from hydroxyl, O-(C₁-C₈ alkyl), NH₂, N(CH₃)₂, and CONH₂), or NH(R¹⁰) (wherein R¹⁰ is C₁-C₈ alkyl, arylalkyl, or heterocyclyl, and these groups are unsubstituted or substituted with one or more groups selected from hydroxyl, O-(C₁-C₈ alkyl), NH₂, N(CH₃)₂, and CONH₂) is a preferred compound, and a compound in which R⁸ is NH(R¹⁰) (wherein R¹⁰ is C₂-C₅ alkyl substituted with one or two hydroxyl, for example, CH₂CH₂OH and CH₂CH(OH)CH₂OH), or NH-C(O)Z (wherein Z is heterocyclyl substituted with one hydroxyl, for example, 4-hydroxy-2-pyrrolidinyl) is a further preferred compound.

More specific examples of preferable compounds among the compounds represented by formula (I) include the following compounds:
(a) a compound
   wherein R¹ is C₃-C₇ alkyl,
   R³ is 1,1,1,3,3,3-hexafluoro-2-hydroxypropan-2-yl,
   R², R⁴, R⁵, R⁶, and R⁹ are hydrogen, and
   at least one of R⁷ and R⁸ is
      NH₂,
      NH-C(O)Z (Z is Ci-Cs alkyl, heterocyclyl, or NH-(Ci-Cs alkyl), which is unsubstituted or substituted with one or more groups selected from hydroxyl and NH₂),
      N(R¹⁰)(R¹¹) (R¹⁰ and R¹¹ are each independently C₁-C₈ alkyl or arylalkyl, which is unsubstituted or substituted with one or more groups selected from hydroxyl, O-(C₁-C₈ alkyl), NH₂, N(CH₃)₂, and CONH₂), or
      NH(R¹⁰) (R¹⁰ is C₁-C₈ alkyl, arylalkyl, or heterocyclyl, which is unsubstituted or substituted with one or more groups selected from hydroxyl, O-(C₁-C₈ alkyl), NH₂, N(CH₃)₂, and CONH₂);
(b) a compound
   wherein R¹ is C₃-C₇ alkyl,
   R³ is 1,1,1,3,3,3-hexafluoro-2-hydroxypropan-2-yl,
   R², R⁴, R⁵, R⁶, R⁷, and R⁹ are hydrogen, and
   R⁸ is
      NH(R¹⁰) (R¹⁰ is C₂-C₅ alkyl substituted with one or two hydroxyl groups, for example, CH₂CH₂OH or CH₂CH(OH)CH₂OH) or
      NH-C(O)Z (Z is heterocyclyl substituted with one hydroxyl group, for example, 4-hydroxy-2-pyrrolidinyl);
(c) a compound
   wherein R¹ is C₄ alkyl,
   R³ is 1,1,1,3,3,3-hexafluoro-2-hydroxypropan-2-yl,
   R², R⁴, R⁵, R⁶, and R⁹ are hydrogen, and
   at least one of R⁷ and R⁸ is
      NH₂,
      NH-C(O)Z (Z is Ci-Cs alkyl, heterocyclyl, or NH-(Ci-Cs alkyl), which is unsubstituted or substituted with one or more groups selected from hydroxyl and NH₂),
      N(R¹⁰)(R¹¹) (R¹⁰ and R¹¹ are each independently C₁-C₈ alkyl or arylalkyl, which is unsubstituted or substituted with one or more groups selected from hydroxyl, O-(C₁-C₈ alkyl), NH₂, N(CH₃)₂, and CONH₂), or
      NH(R¹⁰) (R¹⁰ is Ci-Cs alkyl, arylalkyl, or heterocyclyl, which is unsubstituted or substituted with one or more groups selected from hydroxyl, O-(C₁-C₈ alkyl), NH₂, N(CH₃)₂, and CONH₂); and (d) a compound
   wherein R¹ is C₄ alkyl,
   R³ is 1,1,1,3,3,3-hexafluoro-2-hydroxypropan-2-yl,
   R², R⁴, R⁵, R⁶, R⁷, and R⁹ are hydrogen, and
   R⁸ is NH(R¹⁰) (R¹⁰ is C₂-C₅ alkyl substituted with one or two hydroxyl groups, for example, CH₂CH₂OH or CH₂CH(OH)CH₂OH) or NH-C(O)Z (Z is heterocyclyl substituted with one hydroxyl group, for example, 4-hydroxy-2-pyrrolidinyl).

Specific examples of a further preferable compound among the compounds represented by formula (I) include the following compound:
(e) a compound
wherein R¹ is C₄ alkyl,
R³ is 1,1,1,3,3,3-hexafluoro-2-hydroxypropan-2-yl,
R², R⁴, R⁵, R⁶, R⁷, and R⁹ are hydrogen, and
R⁸ is NH(R¹⁰) (R¹⁰ is C₂-C₃ alkyl substituted with one or two hydroxyl groups, for example, CH₂CH₂OH or CH₂CH(OH)CH₂OH).

Among the compounds represented by formula (I), for example, a compound wherein R¹ is C₄ alkyl, R³ is 1,1,1,3,3,3-hexafluoro-2-hydroxypropan-2-yl, R⁸ is NH₂, and R², R⁴, R⁵, R⁶, R⁷, and R⁹ are hydrogen (NR-31-5) can be produced as follows.

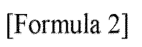

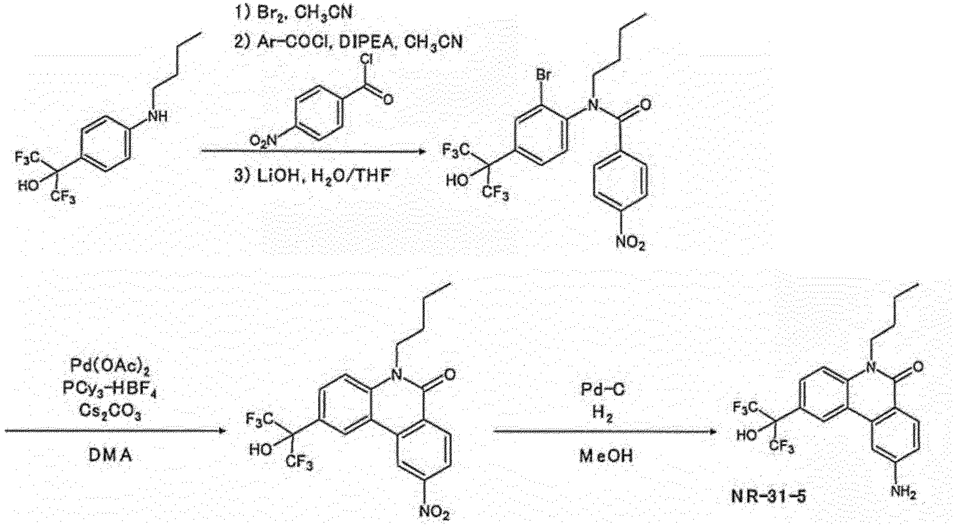

Under argon atmosphere, a bromine/acetonitrile solution is added dropwise to an acetonitrile solution of the compound 10c (NR-31-2), described in Y. Nishiyama, et al., Bioorganic & Medicinal Chemistry 22 (2014) 2799-2808 under ice-cooling. The mixture is agitated at room temperature, and diisopropylethylamine is then added dropwise under ice-cooling. p-Nitrobenzoyl chloride is subsequently added, followed by reaction at room temperature. The reaction solution is poured into iced water, followed by extraction with ethyl acetate. The organic phase is washed with a saturated sodium chloride aqueous solution, the resultant is dried over sodium sulfate, and the drying agent is then filtered off. The filtrate is concentrated to obtain the residue, and tetrahydrofuran and an aqueous solution of lithium hydroxide monohydrate are added to the residue, followed by the reaction at room temperature to obtain an N-(p-nitrobenzoyl)-substituted product (NR-31-3). Under argon atmosphere, the N-(p-nitrobenzoyl)-substituted product is dissolved in N,N-dimethylacetamide, and cesium carbonate, palladium(II) acetate, and tricyclohexylphosphine tetrafluoroborate are added thereto in this order. The reaction solution is agitated while heating to obtain a 9-nitrophenanthridinone derivative (NR-31-4). Under argon atmosphere, the 9-nitrophenanthridinone derivative is dissolved in methanol, palladium-carbon is added thereto, and argon is replaced with hydrogen, followed by reaction at room temperature to obtain a 9-aminophenanthridinone derivative (NR-31-5).

The 9-aminophenanthridinone derivative (NR-31-5) can be modified by N-alkylation, N-acylation, or the like to be converted to various N-substituted products.

In the synthesis process, p-fluorobenzoyl chloride can be used instead of p-nitrobenzoyl chloride to obtain a 9-fluorophenanthridinone derivative, which can then be reacted with amine compounds such as 3-amino-1,2-propanediol to be converted into various N-substituted products.

Further, in the synthesis process, m-nitrobenzoyl chloride can be used instead of p-nitrobenzoyl chloride to obtain an 8-aminophenanthridinone derivative, which can be modified by N-alkylation, N-acylation, or the like to be converted to various N-substituted products.

The product obtained may be purified by a customary method, for example, column chromatography using, e.g., silica gel, as a carrier and a recrystallization method using, e.g., methanol, ethanol, chloroform, dimethyl sulfoxide, n-hexane-ethyl acetate or water. Examples of an elution solvent for column chromatography include methanol, ethanol, chloroform, acetone, hexane, dichloromethane, ethyl acetate and mixed solvents of these.

The compound as mentioned above can be used as an anti-coronavirus drug in combination with a customary pharmaceutical carrier. The dosage form thereof is not particularly limited and appropriately selected and used depending on needs. Examples of the dosage form include oral agents such as a tablet, a capsule, a granule, a fine granule, a powder, a sustained release preparation, a liquid preparation, a suspension, an emulsion, a syrup and an elixir and parenteral agents such as an injection and a suppository.

An oral agent is produced by using, for example, starch, lactose, sucrose, mannitol, carboxymethylcellulose and inorganic salts in accordance with an ordinary method. In addition to these components, e.g., a binder, a disintegrant, a surfactant, a lubricant, a glidant, a flavoring agent, a colorant and/or a perfume can be appropriately added.

Examples of the binder include starch, dextrin, gum arabic, gelatin, hydroxypropyl starch, methylcellulose, sodium carboxymethylcellulose, hydroxypropylcellulose, crystalline cellulose, ethylcellulose, polyvinyl pyrrolidone and macrogol.

Examples of the disintegrant include starch, hydroxypropyl starch, sodium carboxymethylcellulose, calcium carboxymethylcellulose, carboxymethylcellulose and a low-substituted hydroxypropylcellulose.

Examples of the surfactant include sodium lauryl sulfate, soy lecithin, sucrose fatty acid ester and polysorbate 80.

Examples of the lubricant include talc, wax, hydrogenated vegetable oil, sucrose fatty acid ester, magnesium stearate, calcium stearate, aluminum stearate and polyethylene glycol.

Examples of the glidant include light anhydrous silicic acid, dry aluminum hydroxide gel, synthetic aluminum silicate and magnesium silicate.

An injection is produced in accordance with an ordinary method. As a diluent, generally, distilled water for injection, saline, a glucose solution, olive oil, sesame oil, peanut oil, soybean oil, corn oil, propylene glycol, polyethylene glycol, and/or the like can be used. If necessary, a disinfectant, a preservative, a stabilizer, an isotonic agent, a soothing agent, and/or the like may be added. In view of stability, an injection can be added in, e.g., a vial, frozen and subjected to ordinary lyophilization to remove a water content. From the lyophilized injection, a liquid preparation can be prepared again immediately before use. The content of the compound of formula (I) in the injection may be varied between the 5 and 50 wt%; however, the content is not limited to this.

Examples of other parenteral agents include a suppository for intrarectal administration, which can be produced in accordance with an ordinary method.

The administration schedule of an anti-coronavirus drug formulated varies depending on, e.g., the dosage form and the route of administration. For example, the anti-coronavirus drug can be administered once to four times per day in a period from a week to 3 months.

In order to obtain a desired effect, the dose of an oral agent, which varies depending on the age, body weight and severity of a disease of a patient, is usually 0.1 to 6000 mg and preferably 100 to 2000 mg per adult in terms of the weight of the compound of formula (I), for example, and suitably divided into several portions per day and administered.

In order to obtain a desired effect, the dose of a parenteral agent, which varies depending on the age, body weight and severity of a disease of a patient, is usually 0.1 to 6000 mg and preferably 100 to 2000 mg per adult in terms of the weight of the compound of formula (I), for example, and suitably administered by intravenous injection, intravenous drip infusion, subcutaneous injection, or intramuscular injection.

The anti-coronavirus drug of the present invention exhibits antiviral activity against coronaviruses which cause COVID-19, severe acute respiratory syndrome (SARS), Middle East respiratory syndrome (MERS), and the like, and can be used for preventing or treating coronavirus infectious diseases such as COVID-19, SARS, and MERS. The treatment in the present invention also includes the prevention of aggravation thereof.

The compound represented by formula (I) can be incorporated as the only active ingredient in a pharmaceutical composition or incorporated with other active ingredients.

In addition, the compound represented by formula (I) may be used in combination with other agents which are effective against coronavirus infection such as SARS-CoV-2 infection. These agents can be administered separately in the course of a treatment or combined with the compound represented by formula (I) in a single dosage form such as tablets, intravenous solutions and capsules. Examples of these other agents include remdesivir.

It is known that the coronavirus infects various animals, and SARS-CoV also infects various animals beyond species. Accordingly, subjects to be treated by the anti-coronavirus drug of the present invention are not limited to humans, and include various animals such as pets (for example, dogs and cats), pigs, camels, bats, masked palm civets, tigers, ferrets, golden hamsters, minks, and sparrows.

This description includes part or all of the content as disclosed in the description of Japanese Patent Application No. 2021-185802, which is a priority document of the present application.

### Examples

Now, the present invention will be more specifically described below by way of Examples; however, the scope of the present invention is not limited to them.

### [Example 1] Anti-SARS-CoV-2 effect (Assay with HEK293T/ACE2 cells)

### (1) Determination of cell viability (absorbance)

HEK293T/ACE2 cells (2 × 10⁴ cells/well) were plated on microplates together with 100 µL of cell culture solution. After 24 hours of culture, 50 µL of various agents prepared by diluting drug stock solution to 4 times the final concentration was added to each well, and 50 µL of virus solution of SARS-CoV-2 (WK-521, obtained from the National Institute of Infectious Diseases, Japan) was also added with multiplicity of infection (MOI) = 0.1 (plate for infection). To a plate for cells, 50 µL of various agents prepared by diluting drug stock solution to 4 times the final concentration was added to each well, and 50 µL of cell culture solution was also added. After 3 days of culture, culture supernatants of the plate for infection were transferred to a new plate and stored at -80°C. From the plate for cells, 110 µL of culture solution was discarded and 10 µL of Cell Counting Kit-8 was added. After 2 hours of culture in a CO₂ incubator, absorbance was measured at 450 nm (620 nm).

### (2) qPCR measurement of viral RNA in culture supernatants

To 50 µL of culture supernatants, 50 µL of DNA/RNA Shield (Zymo Research Corporation) was added, and RNA extraction was performed (15 µL) using Quick-RNA Viral 96 Kit (Zymo Research Corporation) according to the specification. The extracted RNA was diluted 10-fold using nuclease-free water, which was used as an RNA sample. The RNA sample was used to synthesize cDNA using High-Capacity RNA-to-cDNA^{™} Kit (Thermo Fisher Scientific K.K.) (Table 1). The qPCR measurement was performed under the conditions shown in Table 2.

**[Table 1]**

| | Each reaction | RT reaction | Temp | Time |
|---|---|---|---|---|
| 2X RT Buffer | 10.0µL | Reverse Transcription | 37°C | 60 min |
| 20X RT Enzyme Mix | 1 µL | RT inactivation | 95°C | 5 min |
| Nuclease-free Water | 4 µL | Hold | 4°C | indefinite |
| Total volume RT master mix | 15 µL | | | |
| + RNA sample | 5 µL | | | |
| Final volume | 20 µL | | | |

**[Table 2]**

| | Each reaction | qPCR reaction | Reps | Temp | Time |
|---|---|---|---|---|---|
| 2X TaqMan Gene Expression Master Mix | 12.5 µL | UDG incubation | 1 | 50°C | 2 min |
| Fwd Primer | 0.225 µL | Enzyme activation | 1 | 95°C | 10 min |
| Rev Primer | 0.225 µL | PCR (cycle) | 40 | 95°C | 15 sec |
| TaqMan Probe | 0.625 µL | | | 60°C | 1 min |
| Nuclease-free Water | 6.425 µL | | | | |
| Total volume qPCR master mix | 20 µL | | | | |
| + cDNA sample | 5 µL | | | | |
| Final volume | 25 µL | | | | |

The results of the evaluation of anti-SARS-CoV-2 effect of various phenanthridinone derivatives are shown in Table 3.

**[Table 3]**

| Name | Structure | IC₅₀ (µM) | IC₉₀ (µM) | CC₅₀ (µM) |
|---|---|---|---|---|
| NR-31 | 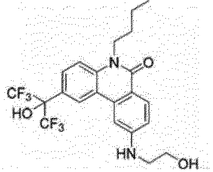 | 0.063 | 1.76 | >20 |
| NR-30 | 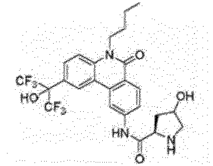 | 0.059 | 1.84 | 13.55 |
| NR-90 | 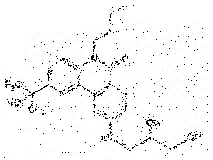 | 0.58 | 3.87 | >20 |
| TK-04 | 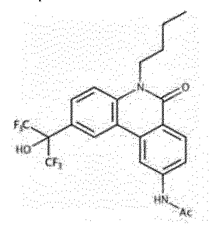 | 0.576 | 5.97 | 7.13 |
| NR-31-5 | 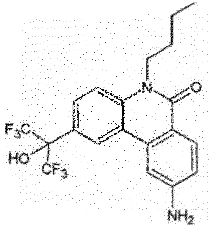 | 0.546 | 2.63 | >20 |
| NR-16 | 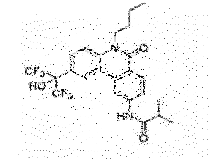 | 0.575 | 2.03 | 15.76 |
| NR-17 | 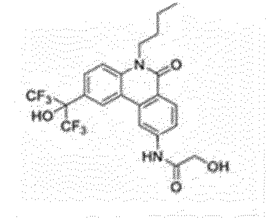 | 0.519 | 3.35 | >20 |
| NR-28 | 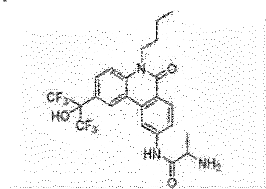 | 0.12 | 1.68 | 11.8 |
| NR-38 | 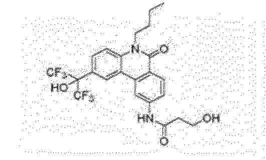 | 0.058 | >20 | >20 |
| NR-44 | 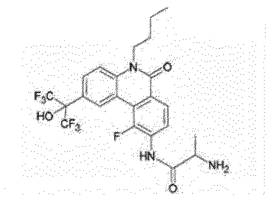 | 0.38 | 3.76 | 10.3 |
| NR-47 | 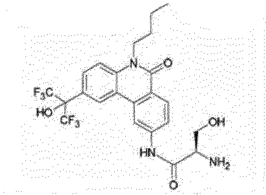 | 0.7 | 3.3 | 11.7 |
| NR-54 | 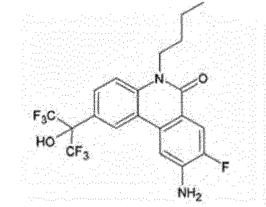 | 0.94 | 4.64 | 27.2 |
| NR-55 | 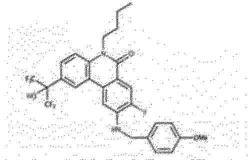 | 0.15 | 6.79 | >80 |
| NR-56 | 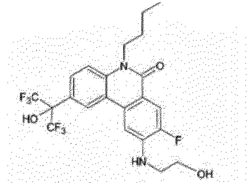 | 0.29 | 8.07 | 29.5 |
| NR-78 | 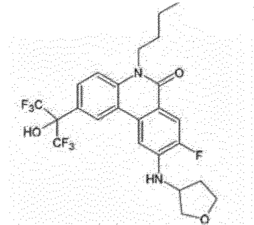 | 0.65 | 3.59 | 17.8 |
| NR-87 | 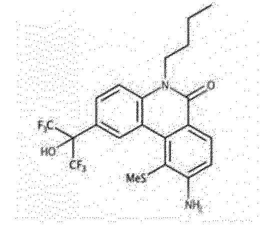 | 0.85 | 4.9 | 18.2 |
| NR-89 | 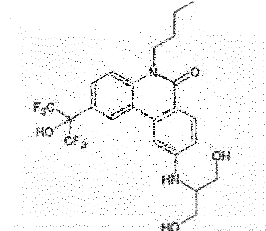 | 0.35 | 6.1 | >20 |
| NR-50 | 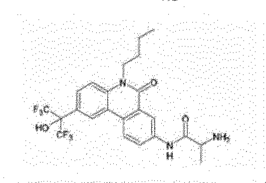 | 0.65 | 4.60 | 12.0 |
| NR-52 | 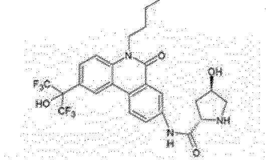 | 0.14 | 2.43 | 18.1 |
| NR-72 | 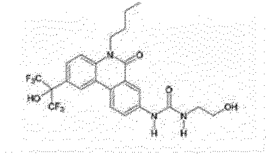 | 0.62 | 2.29 | 9.0 |
| NR-86 | 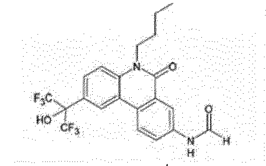 | 0.08 | 3.07 | 16.7 |
| NR-56 | 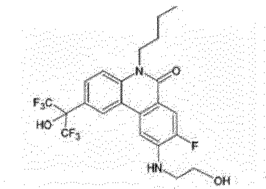 | 0.29 | 8.07 | 29.5 |
| NR-91 | 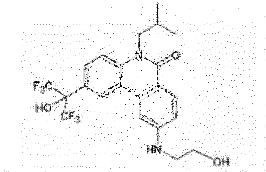 | 0.51 | 4.0 | >20 |
| NR-92 | 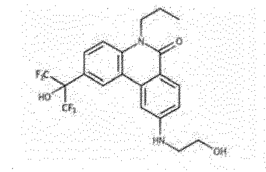 | 0.72 | 3.42 | >20 |
| NR-96 | 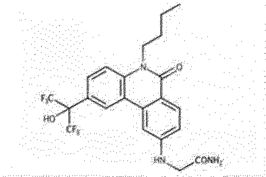 | 0.68 | 4.0 | >20 |
| NR-98 | 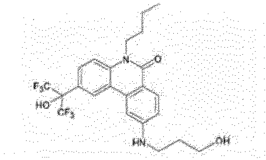 | 1.16 | >10 | >20 |
| NR-99 | 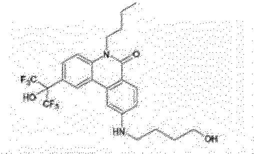 | 1.30 | >10 | >20 |
| NR-100 | 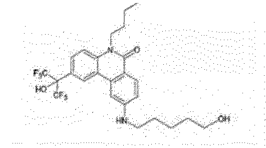 | 0.59 | >10 | >20 |
| NR-101 | 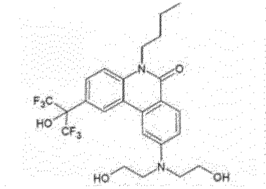 | 0.54 | >10 | >20 |
| NR-102 | 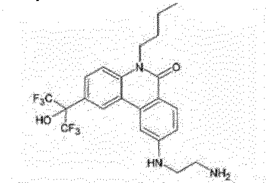 | 0.75 | 2.4 | 5.5 |
| NR-103 | 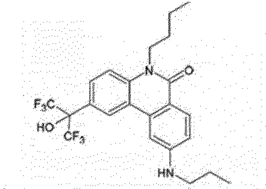 | 2.35 | >10 | >20 |
| NR-105 | 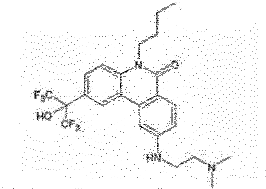 | 1.48 | >10 | 7.4 |

| | | | | |
|---|---|---|---|---|
| IC₅₀: 50% inhibitory concentration (concentration of agent which reduces viral production and replication by 50%) IC₉₀: 90% inhibitory concentration (concentration of agent which reduces viral production and replication by 90%) CC₅₀: 50% cytotoxic concentration (concentration of agent which reduces the number of viable cells by 50%) | | | | |

In Table 3, IC₅₀ and IC₉₀ show the results of qPCR measurement, and CC₅₀ shows the results of the determination of cell viability (absorbance).

Table 3 shows that the phenanthridinone derivatives have the anti-SARS-CoV-2 effect.

### [Example 2] Anti-SARS-CoV effect

IC₅₀ and IC₉₀ were obtained in the same manner as in Example 1 except that SARS-CoV (SARS-CoV-HKU-39849) was used instead of SARS-CoV-2. The results of the evaluation of anti-SARS-CoV effect of various phenanthridinone derivatives are shown in Table 4.

**[Table 4]**

| Name | Structure | IC₅₀ (µM) | IC₉₀ (µM) |
|---|---|---|---|
| NR-31 | 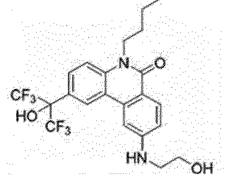 | 0.74 | 6.24 |
| NR-30 | 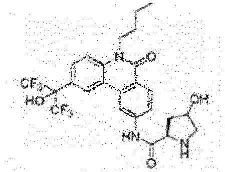 | 6.95 | 19.35 |
| NR-90 | 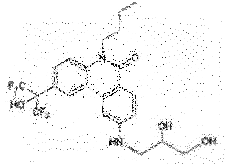 | 2.40 | 14.15 |
| NR-89 | 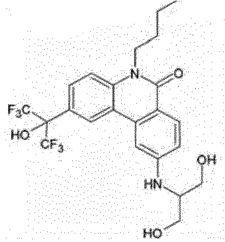 | 4.74 | 20.24 |

### [Example 3] Anti-MERS-CoV effect

IC₅₀ and IC₉₀ were obtained in the same manner as in Example 1 except that MERS-CoV (MERS-CoV-EMC/2012) was used instead of SARS-CoV-2. The results of the evaluation of anti-MERS-CoV effect of various phenanthridinone derivatives are shown in Table 5.

**[Table 5]**

| Name | Structure | IC₅₀ (µM) | IC₉₀ (µM) |
|---|---|---|---|
| NR-31 | 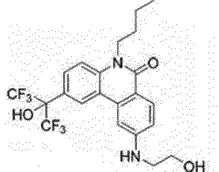 | 2.15 | 5.96 |
| NR-30 | 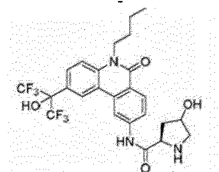 | 12.51 | 21.81 |
| NR-90 | 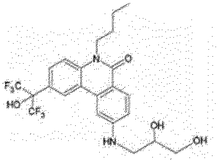 | 3.84 | 13.55 |
| NR-89 | 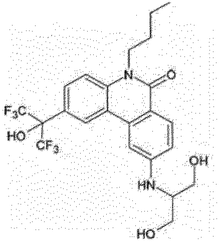 | 6.77 | 19.04 |

### [Example 4] Anti-SARS-CoV-2 (mutant strain) effect (Assay with VeroE6/TMPRSS2 cells)

VeroE6/TMPRSS2 cells highly sensitive to SARS-CoV-2 were plated on microplates together with 100 µL of cell culture solution (2 × 10⁴ cells/well). After 24 hours of culture, 50 µL of NR-31 solution prepared by diluting the drug stock solution to 4 times the final concentration was added to each well, and 50 µL of virus solution of SARS-CoV-2 mutant strains (obtained from the National Institute of Infectious Diseases, Japan) was also added to each well with multiplicity of infection (MOI) = 0.01, and then the cells were cultured at 37°C for 3 days. After the culture, 110 µL of culture supernatants were discarded, and 10 µL of Cell Counting Kit-8 (DOJINDO LABORATORIES, a viable cell counting kit using water-soluble tetrazolium salt WST-8 (2-(2-methoxy-4-nitrophenyl)-3-(4-nitrophenyl)-5-(2,4-disulfophenyl)-2H-tetrazolium monosodium salt) as a coloring reagent) was added. After 2 hours of culture, 100 µL of hydrochloric acid in 2-propanol was added, mixed adequately, and then the supernatant was transferred to fresh microplate, and the absorbance of each well was measured at 450/620 nm. The anti-SARS-CoV-2 effect and cytotoxicity of each agent were determined by comparing the number of viable cells in infected and uninfected cells with those in the absence of the agent.

The evaluation results of the effect of NR-31 against each SARS-CoV-2 strain are shown in Table 6.

**[Table 6]**

| | | |
|---|---|---|
| CC₅₀ (µM) | | 17.6 |
| EC₅₀ (µM) | WK-521 (WT) | 4.4 |
| | TY7-501 (Brazilian strain) | 3.53 |
| | TY7-503 (Brazilian strain) | 5.28 |
| | QK002 (British strain) | 3.37 |
| | QNH001 (British strain) | 4.07 |
| | QNH002 (British strain) | 3.62 |
| | TY8-612 (South African strain) | 5.12 |
| | TY11-927 (Indian strain) | 3.47 |

| | | |
|---|---|---|
| EC₅₀: 50% effective concentration (concentration of agent which inhibits cell death induced by SARS-CoV-2 infection by 50%) CC₅₀: 50% cytotoxic concentration (concentration of agent which reduces the number of viable cells by 50%) | | |

The phenanthridinone derivatives were synthesized as described below.

### (Synthesis Example 1) Synthesis of NR-31-5

### (1) Synthesis ofNR-31-3

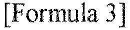

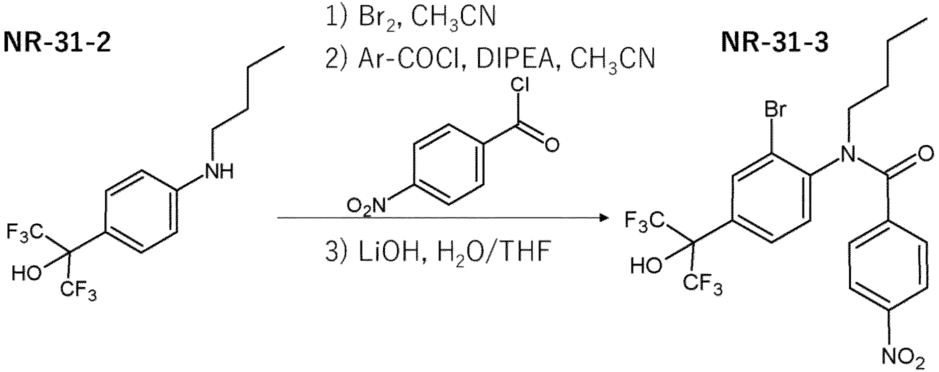

Under argon atmosphere, NR-31-2 (78.5 g, 249 mmol) was added to acetonitrile (393 mL). A bromine (41.8 g, 262 mmol)/acetonitrile (79.0 mL) solution was added dropwise under ice-cooling. The mixture was agitated at room temperature for 1 hour. Under ice-cooling, diisopropylethylamine (89.0 mL, 511 mmol) was added dropwise, and p-nitrobenzoyl chloride (106 g, 573 mmol) was subsequently added in portions. The mixture was agitated at room temperature for 15 hours. The reaction solution was poured into iced water, followed by extraction with ethyl acetate. The organic phase was washed with a saturated sodium chloride aqueous solution, and the resultant was dried over sodium sulfate. The drying agent was filtered off, and the filtrate was concentrated. Tetrahydrofuran (315 mL) and an aqueous solution of lithium hydroxide monohydrate (31.4 g, 748 mmol) (185 mL) were added to the residue, and the mixture was agitated at room temperature for 2 hours. The pH was adjusted to 4 with hydrochloric acid, followed by extraction with ethyl acetate. The organic phase was washed with a saturated sodium bicarbonate aqueous solution and a saturated sodium chloride aqueous solution in this order, and the resultant was dried over sodium sulfate. The drying agent was filtered off, and the filtrate was concentrated. The residue was purified to obtain NR-31-3 (97.5 g) (yield = 72%).

### (2) Synthesis of NR-31-4

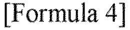

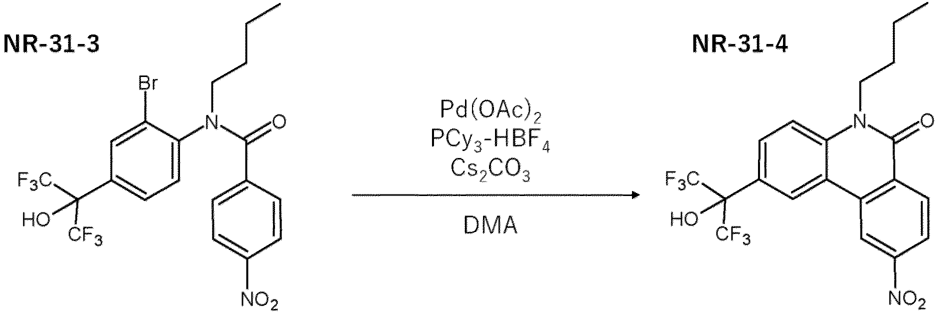

Under argon atmosphere, NR-31-3 (97.5 g, 179 mmol) was added to N,N-dimethylacetamide (1.43 L). Cesium carbonate (263 g, 808 mmol), palladium(II) acetate (8.06 g, 35.9 mmol), and tricyclohexylphosphine tetrafluoroborate (16.5 g, 44.9 mmol) were added to the reaction solution in this order, and the mixture was agitated at 100°C for 45 minutes. The mixture was cooled to room temperature and filtered through celite. The filtrate was poured into city water, and the pH was adjusted to 4 to 5 with hydrochloric acid, followed by extraction with ethyl acetate/heptane. The organic phase was washed with city water and a saturated sodium chloride aqueous solution in this order, and the resultant was dried over sodium sulfate. The drying agent was filtered off, and the filtrate was concentrated. The residue was purified to obtain NR-31-4 (57.4 g) (yield = 69%).

### (3) Synthesis of NR-31-5

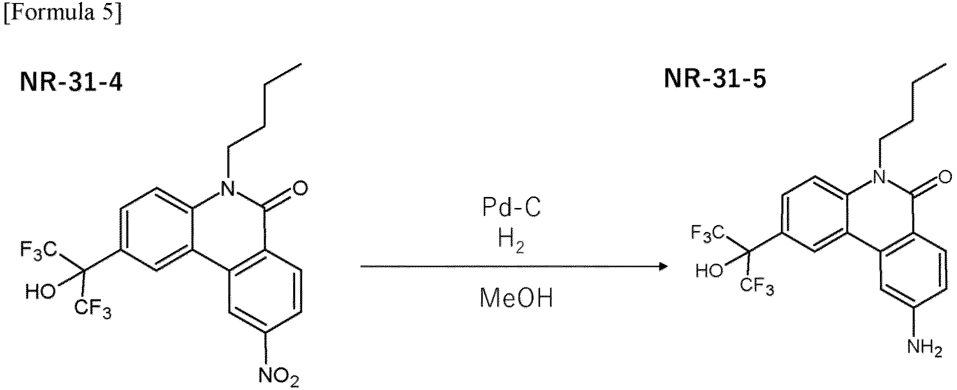

Under argon atmosphere, NR-31-4 (57.4 g, 124 mmol) was added to methanol (859 mL). 5% palladium-carbon (26.4 g, 12.4 mmol, 50% wet product) was added to the reaction solution, and argon within the system was replaced with hydrogen. The mixture was agitated at room temperature for 6 hours and filtered through celite, and the filtrate was concentrated. The residue was purified to obtain NR-31-5 (49.6 g) (yield = 92%).

### (Synthesis Example 2) Synthesis of NR-31

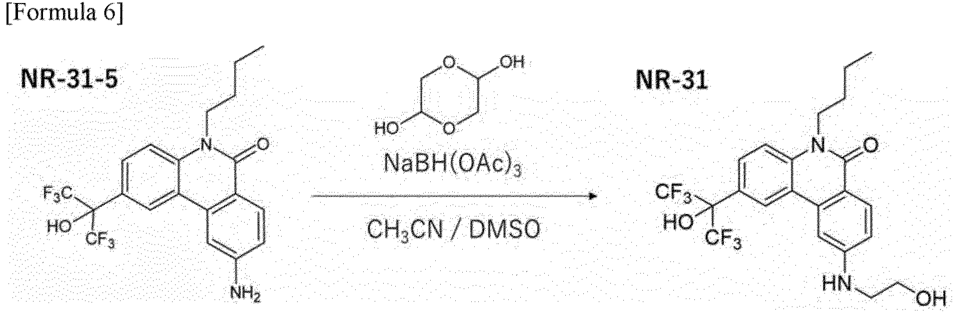

Under argon atmosphere, NR-31-5 (25.0 g, 57.8 mmol) was added to acetonitrile (501 mL) and dimethyl sulfoxide (62.5 mL). Glycolaldehyde dimer (6.25 g, 52.0 mmol) and sodium triacetoxyborohydride (22.1 g, 104 mmol) were added to the reaction solution, and the mixture was agitated at room temperature for 21 hours. The solvent was distilled off, and city water, ethyl acetate, and heptane were added to the residue. The aqueous phase was extracted with ethyl acetate, and the organic phase was washed with city water and a saturated sodium chloride aqueous solution in this order. The organic phase was dried over sodium sulfate, the drying agent was filtered off, and the filtrate was concentrated. The residue was purified to obtain NR-31 (15.7 g).
¹H-NMR (400MHz, CD₃OD) : δ = 8.57 (d, 1H, J = 2.0 Hz), 8.08 (d, 1H, J = 8.8 Hz), 7.77 (d, 1H, J = 8.8 Hz), 7.52 (d, 1H, J = 8.8 Hz), 7.29 (d, 1H, J= 2.0 Hz), 6.87 (dd, 1H, J = 8.8, 2.0 Hz), 4.27-4.31 (m, 2H), 3.71 and 3.34 (each 2H, t, J = 6.0 Hz), 1.70-1.62 (2H, m), 1.46-1.37 (2H, m), 0.92 (3H, t, J = 7.4 Hz)

### (Synthesis Example 3) Synthesis of NR-30

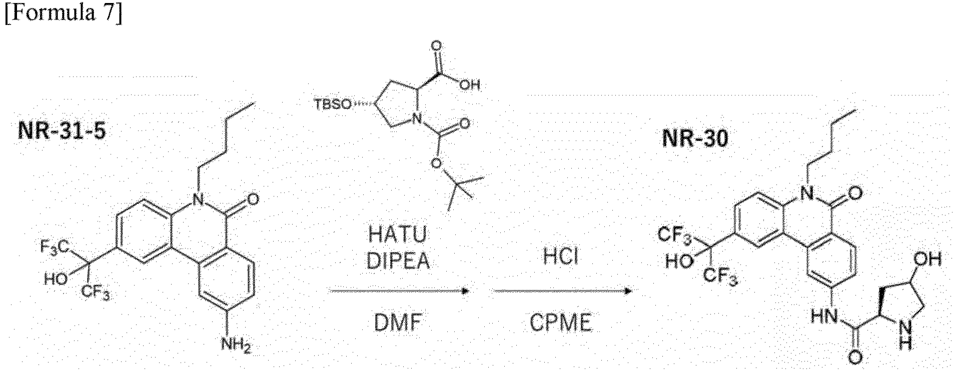

NR-31-5 was subjected to condensation reaction with 1-(tert-butoxycarbonyl)-trans-4-(tert-butyldimethylsilyloxy)-L-proline in the presence of HATU in N,N-dimethylformamide solvent and diisopropylethylamine. The reaction solution was added to water, and the aqueous phase was extracted with ethyl acetate. The organic phase was washed with city water and a saturated sodium chloride aqueous solution in this order. The organic phase was dried over sodium sulfate, the drying agent was filtered off, and the filtrate was concentrated. The residue was dissolved in cyclopentyl methyl ether, and the solution was treated with hydrochloric acid to be subjected to deprotection reaction. The reaction solution was neutralized with a saturated sodium bicarbonate aqueous solution, and the aqueous phase was extracted with ethyl acetate. The organic phase was washed with city water and a saturated sodium chloride aqueous solution in this order. The organic phase was dried over sodium sulfate, the drying agent was filtered off, and the filtrate was concentrated. The residue was purified to obtain NR-30.
¹H-NMR (400MHz, CD₃OD) : δ = 8.89 (d, 1H, J = 2.0 Hz), 8.74 (s, 1H), 8.38 (d, 1H, J = 8.8 Hz), 7.94 (d, 1H, J = 8.8 Hz), 7.83 (dd, 1H, J= 2.0, 8.8 Hz), 7.68 (d, 1H, J = 8.8 Hz), 4.41-4.45 (m, 3H), 4.11 (t, 1H, J = 8.4 Hz), 3.13 (dd, 1H, J = 6.0, 2.0Hz), 2.97-3.00 (m, 1H), 2.25-2.30 (m, 1H), 2.00-2.05 (m, 1H), 1.74-1.82 (m, 2H), 1.48-1.57 (m, 2H), 1.02 (t, 3H, J = 7.4 Hz)

### (Synthetic Example 4) Synthesis of NR-16

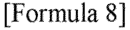

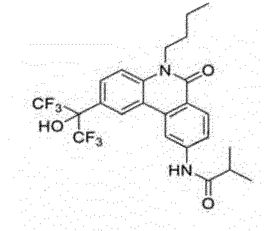

Dichloromethane and N,N-diisopropylethylamine were added to NR-31-5 and isobutyryl chloride, followed by reaction in the presence of methanol and potassium carbonate to obtain NR-16.

### (Synthesis Example 5) Synthesis of NR-90

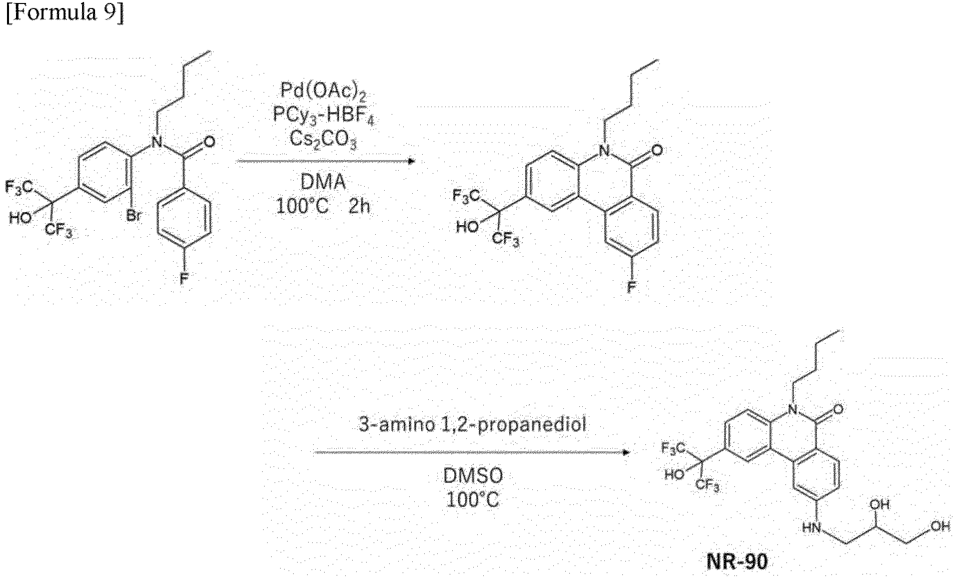

9-fluorophenanthridinone derivative was obtained in the same manner as in Synthesis Example 1, (2) Synthesis of NR-31-4, except that N-(p-fluorobenzoyl)-substituted product was used instead of N-(p-nitrobenzoyl)-substituted product (NR-31-3), and 3-amino-1,2-propanediol was then reacted therewith in dimethyl sulfoxide at 100°C to obtain NR-90.
¹H-NMR (400MHz, CD₃OD) : δ =8.68 (d, 1H, J = 2.0 Hz), 8.17 (d, 1H, J = 8.8 Hz), 7.87(d, 1H, J = 8.8 Hz), 7.61 (d, 1H, J = 8.8 Hz), 7.41 (s, 1H), 6.99 (dd, 1H, J = 2.0, 8.8 Hz), 4.37-4.41 (m, 2H), 3.88-3.94 (m, 1H), 3.60-3.68 (m, 2H), 3.47-3.52 (dd, 1H, J = 5.2, 13.2 Hz), 3.27-3.35 (m, 1H), 1.72-1.79 (m, 2H), 1.48-1.56 (m, 2H), 1.02 (t, 3H, J = 7.4 Hz)

### (Synthesis Example 6)

Other compounds described in Table 3 were synthesized according to Synthesis Examples 1 to 5.

All publications, patents, and patent applications cited herein are incorporated herein by reference in their entirety.

## Claims

1. A compound represented by formula (I), a pharmaceutically acceptable salt thereof, a solvate thereof, or a prodrug thereof 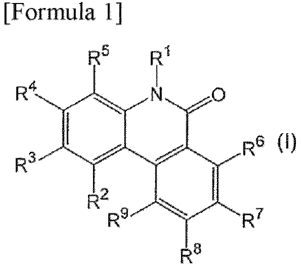 wherein,
R¹ is selected from C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from halogen, hydroxyl, NH₂, NO₂, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)); and
R² to R⁹ are each independently selected from Substituent group A consisting of hydrogen, halogen, hydroxyl, NH₂, NO₂, OSO₃H, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), NH-C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), NH-C(NH)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(Ci-Cs alkyl)), N(R¹⁰)(R¹¹) (wherein R¹⁰ and R¹¹ are each independently C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl), NH(R¹⁰) (wherein R¹⁰ is Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl), Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl) and Q-(heteroarylalkyl) (wherein Q is O, NH, or S), Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from halogen, hydroxyl, NH₂, NO₂, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), NH-C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), N(R¹⁰)(R¹¹) (wherein R¹⁰ and R¹¹ are each independently C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl), Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)),
provided that any two of R² to R⁵ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from halogen, hydroxyl, NH₂, NO₂, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)),
at least one of R⁷ and R⁸ is NH₂, NH-C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), NH-C(NH)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), N(R¹⁰)(R¹¹) (wherein R¹⁰ and R¹¹ are each independently C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl), or NH(R¹⁰) (wherein R¹⁰ is Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl) (each of these groups being independently unsubstituted or substituted with one or more groups selected from halogen, hydroxyl, NH₂, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), NH-C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, NH₂, or NH-(C₁-C₈ alkyl)), N(R¹⁰)(R¹¹) (wherein R¹⁰ and R¹¹ are each independently C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, arylalkyl, or heterocyclyl), Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)).

2. The compound according to claim 1, a pharmaceutically acceptable salt thereof, a solvate thereof, or a prodrug thereof, wherein at least one of R⁷ and R⁸ is NH₂, NH-C(O)Z (wherein Z is Ci-Cs alkyl, heterocyclyl, or NH-(C₁-C₈ alkyl), and these groups are unsubstituted or substituted with one or more groups selected from hydroxyl and NH₂), N(R¹⁰)(R¹¹) (wherein R¹⁰ and R¹¹ are each independently C₁-C₈ alkyl or arylalkyl, and these groups are unsubstituted or substituted with one or more groups selected from hydroxyl, O-(C₁-C₈ alkyl), NH₂, N(CH₃)₂, and CONH₂), or NH(R¹⁰) (wherein R¹⁰ is C₁-C₈ alkyl, arylalkyl, or heterocyclyl, and these groups are unsubstituted or substituted with one or more groups selected from hydroxyl, O-(C₁-C₈ alkyl), NH₂, N(CH₃)₂, and CONH₂).

3. The compound according to claim 1, a pharmaceutically acceptable salt thereof, a solvate thereof, or a prodrug thereof, wherein R⁸ is NH(R¹⁰) (wherein R¹⁰ is C₂-C₅ alkyl substituted with one or two hydroxyl), or NH-C(O)Z (wherein Z is heterocyclyl substituted with one hydroxyl).

4. The compound according to any one of claims 1 to 3, a pharmaceutically acceptable salt thereof, a solvate thereof, or a prodrug thereof, wherein R¹ is C₃-C₇ alkyl.

5. The compound according to any one of claims 1 to 3, a pharmaceutically acceptable salt thereof, a solvate thereof, or a prodrug thereof, wherein R¹ is C₄ alkyl.

6. The compound according to any one of claims 1 to 5, a pharmaceutically acceptable salt thereof, a solvate thereof, or a prodrug thereof, wherein R³ is 1,1, 1,3,3,3-hexafluoro-2-hydroxypropan-2-yl.

7. An anti-coronavirus drug comprising, as an active ingredient, the compound according to any one of claims 1 to 6, a pharmaceutically acceptable salt thereof, a solvate thereof, or a prodrug thereof.

8. The anti-coronavirus drug according to claim 7, which is an anti-SARS-CoV-2 drug.

9. The anti-coronavirus drug according to claim 8, which is used for preventing and/or treating COVID-19.
